Europäisches Patentamt

European Patent Office

Office européen des brevets

(11) Publication number : **0 521 605 A2**

## (12) EUROPEAN PATENT APPLICATION

(21) Application number : 92304375.6

(22) Date of filing : 14.05.92

(51) Int. Cl.⁵ : **C09D 4/00**

(30) Priority : 16.05.91 US 700779

(43) Date of publication of application :
07.01.93 Bulletin 93/01

(84) Designated Contracting States :
AT BE CH DE DK ES FR GB IT LI NL SE

(71) Applicant : IOPTEX RESEARCH INC.
15715 Arrow Highway
Irwindale, California 91706-2094 (US)

(72) Inventor : Li, Cheng
1238 South Berendo Street, Apt. No. 1
Los Angeles, California 90006 (US)
Inventor : Colvin, Michael
25001 Pacific Coast Hwy.
Malibu, California 90265 (US)
Inventor : Gupta, Amitava
1606 Virginia Road
San Marino, California 91108 (US)

(74) Representative : Cole, William Gwyn, Dr.
Smith & Nephew p l c Corporate Patents and
Trade Marks Dept. Gilston Park
Harlow Essex CM20 2RQ (GB)

(54) **Biocompatible lubricious grafts.**

(57)   A composition suitable for photografting composed of a hydrophilic monomer and a hydrophobic monomer which is either a perfluoroacrylate or a perfluoromethacrylate, which may be used in the manufacture of articles intended to contact moist body surfaces.

EP 0 521 605 A2

Jouve, 18, rue Saint-Denis, 75001 PARIS

The present invention relates to polymer materials and to products formed therefrom. More particularly the present invention relates to polymeric coatings and their use in the manufacture of articles which are intended to contact moist body surface. Such articles include surgical and medical examination gloves and lenses such as contact and intraocular lenses.

As an alternative to the use of lubricants such as powdered starch to improve hand donning properties of surgical gloves, a permanent thin layer of a polymer may be chemically bonded on the surface. The layer should be able to improve dry and wet donning and absorb sweat for greater comfort. The layer also must be non-harmful to the human biological system.

US Patent no. 3813695 describes a surgical glove consisting of an outer layer of flexible material, for example rubber, and an inner layer of hydrophilic material such as hydrogel polymer, the inner and outer layers being bonded together.

It has been found that while hydrophilic coatings on flexible rubber articles, such as surgical gloves, provide a certain degree of lubricity to the skin which contacts with the surface of such gloves, this lubricity is only effective with respect to dry skin and is not present when the user's skin is damp or moist. Most surgeons prefer to don their gloves when their hands are not fully dry. In order to overcome these problems the present invention seeks to provide coatings which provide good lubricity with respect to both dry and damp skin.

Contact and intraocular lenses are often made of relatively hydrophobic materials such as polymethylmethacrylate (PMMA) and lenses of such materials may cause some discomfort to the wearer or patient. It has also been found that by providing a coating comprising both hydrophobic and hydrophilic materials on such lenses the lenses are more comfortable to wear. Intraocular lenses may cause inflammation of ocular tissue inside the eye, leading to long term complications, such as uveitis. Coatings have been developed for intraocular lenses which reduce cellular adhesion and tissue damage upon repeated contact. For example, US Patent no. 4655770 describes a fluorocarbon coating on a medical implant designed to reduce surface energy and hence cellular adhesion.

In accordance with the present invention, there is provided a polymerisable grafting composition comprising monomer or a combination of monomers.

By the term hydrophilic monomer is meant a monomer which when polymerised will form a polymer which will absorb at least 5% by weight of water when immersed in water at 25°C for 24 hours. By the term hydrophobic monomer is meant a monomer which when polymerised will form a polymer which will resist spread of water on its surface, so that the water contact angle (measured by the advancing meniscus method) is greater than 45°. Hydrophilic or hydrophobic coatings have been developed for intraocular and contact lenses. Hydrophilic coatings impart lubricity in the wet state and less adherence to cellular membranes, while hydrophobic coatings are characterised by low surface energy, hence lesser adhesion to proteins. While both types of coatings have their advantages, true biocompatibility requires a combination of hydrophilic and hydrophobic properties on the surface of the intraocular or contact lenses. We have developed several biocompatible graft compositions which have both hydrophilic an hydrophobic properties which can impart lubricity in both the dry and wet state.

The coating composition may comprise more than one hydrophilic monomer.

The coating composition may also comprise oligomers, ie. low molecular weight polymers terminated with one or more polymerisable groups.

The present invention also provides an article having a coating on a surface thereof a polymer composition comprising residues derived from a hydrophilic monomer and residues derived from a perfluoroacrylate or a perfluoromethacrylate in proportions sufficient to provide lubricity when in contact with either damp or dry skin.

In accordance with a further embodiment of the invention there is provided a process for the manufacture of coated articles which comprises coating a surface of an article with a polymerisable composition comprising a hydrophilic monomer and a perfluoroacrylate or perfluoromethacrylate and thereafter irradiating the coated composition in order to copolymerise the monomeric components.

Preferably the coated polymerisable composition is subjected to ultraviolet (UV) irradiation to effect polymerisation. More suitably the surface upon which the polymerisable composition is coated is a substrate onto which the polymerisable coating composition may be graft polymerised. Suitable substrates for grafting include natural rubber, preferably lightly vulcanised natural rubber, and acrylic resins such as polymethylmethacrylate.

Suitable hydrophilic monomers for use in the present invention include acrylamide, hydroxyl ethyl methacrylate, 2-hydroxypropyl methacrylate, 2-hydroxypropyl acrylate, hydroxyethyl acrylate and vinyl pyrrolidone.

The coating composition may comprise one or more hydrophilic monomers together with the perfluoro acrylate or methacrylate and optionally other hydrophobic monomer such as methylmethacrylate. The molar ratio of hydrophilic monomers to all hydrophobic monomers (including the perfluoro acrylates or methacrylates) is preferably at least 2:1. Generally the molar ratio of hydrophilic to hydrophobic components need not exceed 1:10. Suitably the ratio of hydrophilic to hydrophobic components is in the range of from about 1:1 to about 1:5.

Suitable perfluoro components for use in the present invention include compounds having the following formula and designations and are available from Minnesota Mining and Manufacturing Company Inc.

| <u>Designation</u> | <u>Formula</u> |
|---|---|
| FX13 | $C_8F_{17}SO_2N(C_2H_5)CH_2CH_2OCOCH=CH_2$ |
| FX14 | $C_8F_{17}SO_2N(C_2H_5)CH_2CH_2OCOC(CH_3)=CH_2$ |
| FX189 | $C_8F_{17}SO_2N(C_4H_9)CH_2CH_2OCOCH=CH_2$ |
| L9911 | $C_4F_9OCF(CF_3)CF_2OCF(CF_3)CH_2OCOCH=CH_2$ |
| L12044 | $C_4F_9OCF(CF_3)CF_2OCF(CF_3OCH_2OCOC(CH_3)=CH_2$ |
| L12043 | $[CH_2-CHOCOCH_2CF(CF_3)O\{CF(CF_3)CF_2O\}_2C_2F_4]_2$ |
| L9367 | $CH_2-CHOCOCH_2-(C_2F_4O)_M-(CF_2O)_nCH2OCOCH=CH_2$ |

The hydrophilic components, such as polyacrylamide, polyhydroxyethyl methacrylate, polyvinyl pyrrolidone and polyhydroxy ethylacrylate in the grafted layer, give superior lubricity with respect to dry skin. The hydrophobic components, such as the perfluoro acrylate and methacrylate polymers exemplified above substantially improve the lubricity with respect to damp skin.

The coating composition may also comprise low to medium molecular weight polyethylene oxide (PEO), molecular weight range 300-2000, terminated with one or two acrylate or methacrylate groups. Such PEO oligomers have a combination of hydrophilic and hydrophilic properties.

It has been found that several combinations of hydrogel monomers and perfluoro acrylate and perfluoro methacrylate monomers, which are grafted onto the surface of the rubber articles, such as surgical gloves, provide good lubricity with respect to both dry and damp skin. According to the present invention, these preferred combinations are hydroxyethyl methacrylate with perfluoro acrylate or perfluoro methacrylate monomers and polyvinyl pyrrolidone monomers.

The monomer combinations are grafted by any suitable radiation source, preferably by a UV irradiation graft technique, more preferably using high intensity UV lamps.

One class of articles according to the present invention is preferably made of a rubber, suitably a natural rubber. The article may be formed (prior to grafting of the hydrophilic and hydrophobic layer) by dipping of a suitable latex. The hydrophilic and hydrophobic layer is grafted onto the surface of the rubber after curing or vulcanisation. The monomer solution, which preferably contains a UV initiator, is applied by dipping the rubber sample in the solution.

Rubber articles, such as surgical gloves, can be produced by the following steps:

(1) washing the rubber sample with isopropanol (IPA),

(2) dipping the sample into a solution consisting of hydrophilic and hydrophobic monomers, UV initiator and solvents;

(3) grafting the hydrophilic and hydrophobic monomers by UV irradiation;

(4) washing the finished rubber sample with IPA; and

(5) drying the sample in air.

Other articles which may be in accordance with the present invention can include other skin- or tissue-containing flexible rubber articles, such as condoms, examination gloves, catheters, ureters, sheets and sheath articles.

More generally, the overall performance of the materials used in biomedical applications depends simultaneously upon satisfactory physical and mechanical properties and compatibility with the biological system involved. The physical and mechanical properties of the material is generally governed by bulk properties, whereas biological compatibility is controlled by the surface chemistry and morphology. Therefore, this invention can be used to improve the surface properties of other classes of body contacting articles such as prostheses, and in particular intraocular lenses and contact lenses.

In order to fully understand the present invention, the following Examples are given by way of illustration only.

## Example 1

A natural rubber latex sample was washed with isopropanol (IPA). Then the washed sample was soaked in an IPA and hexane solution of hydroxyethyl methacrylate and FX13 monomers in a molar ratio of 1 to 1 at 20% concentration and 0.5% UV initiator (Irgacure 184) for 30 to 60 minutes. The soaked rubber sample was treated with high intensity UV radiation generated by high or medium mercury arc lamp for 5 to 30 minutes. The treated rubber was washed with IPA and then air dried.

The grafted rubber latex sample was characterised using the contact angle measurement, FTIR-ATR, SEM, and ESCA. The performance of the grafted rubber sample with respect to dry and wet skin was evaluated by the dry and wet friction measurements. FTIR-ATR analysis showed that the grafting materials were chemically bonded to the surface of the rubber sample. ESCA study confirmed this and indicated that there was 20 to 30% atom percent fluorine on the surface.

## Examples 2 to 22

Example 1 was repeated except that the monomers and solvents were replaced by the ones shown in Table I.

## Table I

### Molar Ratios

| Example 1 | Monomer | of Monomers | Solvents |
|---|---|---|---|
| 2 | HEMA/FX13 | 1/2 | IPA/Hexane |
| 3 | HEMA/FX13 | 1/3 | IPA/Hexane |
| 4 | HEMA/FX13 | 1/4 | IPA/Hexane |
| 5 | HEMA/FX13 | 1/5 | IPA/Hexane |
| 6 | HEMA/FX13 | 1/2 | IPA/CHC13 |
| 7 | HEMA/FX13 | 1/2 | IPA/Toluene |
| 8 | HEMA/FX13 | 1/2 | Acetone/Hexane |
| 9 | HEMA/EHA | 1/3 | IPA/Hexane |
| 10 | HEMA/FX189 | 1/1 | Acetone/Hexane |
| 11 | HEMA/FX189 | 1/1 | Acetone/Hexane |
| 12 | HEMA/FX189 | 1/2 | Acetone/Hexane |
| 13 | HEMA/FX189 | 1/3 | Acetone/Hexane |
| 14 | HEMA/FX189 | 1/4 | Acetone/Hexane |
| 15 | HEMA/FX189 | 1/5 | Acetone/Hexane |
| 16 | NVP/FX189 | 1/1 | $IPA/CHC1_3$ |
| 17 | NVP/FX189 | 1/2 | $IPA/CHC1_3$ |
| 18 | NVP/FX189 | 1/3 | $IPA/CHC1_3$ |
| 19 | NVP/FX189 | 1/4 | $IPA/CHC1_3$ |
| 20 | NVP/FX189 | 1/5 | $IPA/CHC1_3$ |
| 21 | HEMA/L9911 | 1/2 | Acetone/Hexane |
| 22 | NVP/L9911 | 1/2 | $IPA/CHC1_3$ |

The dry and wet friction of the grafted rubber samples were significantly improved. One of the examples is shown in Table II.

## TABLE II

| Sample | State | Coefficient of Friction | slick/slip |
|---|---|---|---|
| Control | Dry | 1.76 | ±0.69 |
| Grafted | Dry | 0.39 | ±0.01 |
| Control | Wet | 1.30 | ±1.30 |
| Grafted | Wet | 0.51 | ±0.02 |

The following methods illustrate the grafting of polymethyl methacrylate (PMMA) with a hydrophilic monomer (eg. N-vinyl pyrrolidone) via UV radiation to improve surface properties. As the hydrophilic monomers are grafted on the hydrophobic PMMA substrate, the contact angle of the substrate drops significantly. Thus, contact angle goniometry is used to characterise the grafted PMMA. In addition, ATR and ESCA were also used to characterise the grafted PMMA.

Method A - Vapour Phase Technique

2M of NVP and 0.2M initiator were mixed in a solvent. The initiators were irgacure 184 or benzophenone. The solvents used were acetone or ethanol. Approximately 100ml of the monomer solution were transferred into a crystallisation dish and placed on the bottom of the reactor chamber. The PMMA samples to be grafted were placed on a sample holder with the polished side face up in the reactor chamber.

The chamber was closed tightly and purged with nitrogen gas for 10 to 30 minutes to ensure there was no air in the reactor system. In the meantime, the reactor chamber was heated up to about 60°C. Once the temperature inside the chamber reached the desired temperature ($\sim$60°C), the UV lamp was turned on and shined directly onto the sample for a specific period of time (eg. 5, 10, 20 or 30 minutes). In some cases, the time for UV exposure was intermittent and in other cases, it was uninterrupted.

The PMMA samples were removed and soaked in IPA for about one hour to remove residual monomer (if any). They were dried in a pre-heated vacuum oven ($\sim$70°C) overnight and the contact angle was measured the next day. The control samples were also prepared in the same manner, except that the solution did not contain NVP monomer.

The following results were observed.

1. There was not significant difference in contact angle of a sample which was exposed to UV light continuously as compared to those exposed to UV light intermittently, for the same time interval. For example, set A results revealed similar contact angle for 5 minutes UV exposure and for 20 minutes UV exposure (at 5 minute intervals).

2. The 30 minute UV exposure with a 15 minute cycle seemed to yield a significant change in the contact angle of the PMMA substrate. However, the results were not reproducible and varied with a large range from 16.2° to 66°. The surfaces of PMMA which showed low contact angles looked bumpy and had a considerable amount of crazing, which at this stage, may have been due to the drying process. ESCA revealed about 6% nitrogen content on the surface and ATR spectrum exhibited a shoulder peak at 1700cm$^{-1}$. Care should be taken to control the correct amount of vapour of monomer in the reactor chamber and to ensure there is sufficient monomer vapour condensed on the surface of PMMA substrate.

Method B1 - Dip-in Technique

Different concentrations of NVP were prepared in isopropyl alcohol with Irgacure 184 as initiator. The solutions used were:

```
22% NVP + 18.6% irgacure 184 in ETOH

10% NVP +  0.1% irgacure 184 in IPA

 5% NVP +  0.1% irgacure 184 in IPA
```

The percentage of irgacure was based on the weight of NVP monomer only.

PMMA samples were soaked in the above solutions for about 30 minutes and exposed to UV light for 15 minutes. The samples were soaked in IPA for one hour to wash off residual monomer and dried in a vacuum oven at 70°C overnight. Contact angle of each sample was checked the next day. The control samples were treated in the same manner except that the solution used contained no NVP monomer.

Method B1 produced contact angle measurements consistent with the concentration of NVP monomer used The contact angle of the treated surface decreased as the concentration of NVP was increased in the solution.

When using 10% NVP for grafting, (lot #99-41), ESCA showed that the surface of grafted PMMA had an atom percentage of 5% nitrogen, which the ATR spectrum showed as a distinct peak at the 1700cm$^{-1}$ region. While thicker coatings were observed on the surface of PMMA substrate, and the coating was occasionally non-uniform, this method is faster and yields more consistent results than Method A.

Table III sets forth the contact angles measured for the sample produced by the vapour phase method. Table IV sets forth the contact angles measured for dip-in method B1.

## TABLE III

CONTACT ANGLE OF NVP GRAFTED PMMA VIA THE VAPOR PHASE UV
IRRADIATION

A. 2M NVP + 0.2M Irgacure 184 in acetone.

| CURING TIME (MIN) | CURING CYCLE | CONTACT ANGLE | LOT # |
|---|---|---|---|
| 5 | 1 x 5 MIN. | 65 ± .71° | 99-13 |
| 10 | 1 X 5 MIN. | 67.9 ± 2.6 | 99-33 |
|  | 1 X 5 MIN. | 69.2 ± 0.45 | 99-15 |
|  | 1 X 5 MIN | 64.7 ± 1.25 | 99-53 |
| 20 | 4 X 5 MIN. | 64.7 ± 1.5 | 99-35 |
|  | 2 X 10 MIN. | 64.4 ± 1.6 | 99-51 |
| 30 | 2 X 15 MIN. | 16.16 ± 2.8 | 99-39 |
|  | 2 X 15 MIN. | 47 ± 17.6 | 99-55 |
|  | 2 X 15 MIN. | 57.8 ± 8.3 | 99-57 |
|  | 2 X 15 MIN. | 61 ± 10 | 99-63 |
|  | 2 X 15 MIN. | 66 ± 3.1 | 99-65 |

B. 2M NVP + 0.2M Benzophenone in acetone.

| CURING TIME (MIN) | CURING CYCLE | CONTACT ANGLE | LOT # |
|---|---|---|---|
| 30 | 2 X 15 MIN. | 55.4 ± 13 | 99-61 |

C. 2M NVP + 0.2M Irgacure 184 in ETOH

| CURING TIME (MIN) | CURING CYCLE | CONTACT ANGLE | LOT # |
|---|---|---|---|
| 5 | 1 X 5 MIN | 64.4 ±.55 | 99-23 |
| 15 | 3 X 5 MIN. | 67.1 ±.89° | 99-25 |
| 20 | 4 X 5 MIN. | 70.4 ±.89° | 99-27 |
| | 4 X 5 MIN | 67.6 ±.65° | 99-29 |
| | 4 X 5 MIN | 70 ± 2.24 | 99-31 |

**D. Control: 0.2M Irgacure 184 in Acetone**

| CURING TIME (MIN) | CURING CYCLE | CONTACT ANGLE | LOT # |
|---|---|---|---|
| 30 | 2 X 15 MIN. | 66 ± 2.55 | 99.43 |

**TABLE IV**

**CONTACT ANGLE OF NVP GRAFTED PMMMA VIA DIP-IN UV IRRADIATION**

| NAME OF SOLUTION | CONTACT ANGLE | LOT # |
|---|---|---|
| 22% NVP + 0.18% IRGACURE IN ETOH | 21.6 ± 6.07° | 99-37 |
| 10% NVP + 0.1% IRGACURE IN IPA | 40.8 ± 12.8 | 99-41 |
| 5% NVP + 0.1% IRGACURE IN IPA | 61.6 ± 7.5<br>44.8 ± 12.15 | 99-45<br>99-49 |
| CONTROL: .005% IRGACURE IN IPA | 68.9 ± 1.45 | 99-47 |
| CONTROL: NO SOLUTION | 64.4 ± 2.6 | 99-47 |

Method B2 - Dip-in Technique

Different concentrations of monomers, or polymers were prepared in isopropyl alcohol and irgacure 184 as initiator. For HEMA, the solvent used was a mixture of acetone and IPA (4:6) so the HEMA monomer can easily penetrate into the PMMA substrate.

PMMA or PMUV buttons were dipped into the monomer or polymer solution for 30 minutes. They were placed on a glass slide with the polished surface face up and were UV irradiated for 15 minutes under a hood. The irradiated samples were then soaked in IPA for 30 minutes to remove residual monomer/polymers. They were dried in a vacuum oven (at 70°C) overnight. The samples were removed the next day and let cool to room temperature before being analysed with contact angle goniometer, FTIR and light microscopy.

The following results were observed.

1. NVP usually produced a thick coating on the surface. Significant crazing of the surface was observed. The contact angle changed from 66° to 40° as the NVP concentration was increased from 5% to 20%. FTIR analysis showed a distinct peak at 1700cm$^{-1}$.

2. Grafting of HEMA appeared to yield a smooth surface. At high concentrations (80% HEMA) some pits were observed on substrate surface. The contact angle of the sample was about 54° when grafting with 80% HEMA concentration. Since water contact angle of polyHEMA is only about 59-60°, the maximum change in contact angle for a grafted PMUV can only be approximately 15°. FTIR revealed a small OH peak at 3400cm$^{-1}$ .

3. PMMA or PMUV grafted with glyceryl monomethacrylate (GMMA) produced a smooth surface. Some pits were also observed on the surface under the microscope. Water contact angle ranged from 64° to 40° for a GMMA concentration range from 10% to 80%. However, the IR spectrum did not exhibit a significant difference as compared to PMUV or PMMA.

4. A series of ten photografting runs was carried out with polyethylene oxide polymers terminated at both ends with acrylate and methacrylate groups. Polyethylene oxide chains of molecular weights 210, 252, 259 and 1000 were used in these runs. Methanol and isopropanol were used as solvent. The concentration of the monomer ranged from 10% to 80% v/v. Table V shows typical data obtained in these runs.

**TABLE V**

| MONOMER/ CONCENTRA- TION | SUBSTRATE | SOAKING/PHOTO- CURING WASHING TIME (min) | THICKNESS OF GRAFT (MICRONS) | CONTACT ANGLE | COMMENTS |
|---|---|---|---|---|---|
| PEGDA/60% (259) | PMUV BUTTONS | 60/5/60 | 100 | 62±1 | 90% COATED; SURFACE SMOOTH AND UNIFORM |
| PEGDMA/20% (252) | PMUV BUTTONS | 60/8/30 | 10 | 45-50 | 100% COATED; SURFACE SMOOTH AND UNIFORM |
| PEGDMA/20% (252) | PMUV | 30/8/30 | 10 | 45-50 | 100% COATED; SURFACE SMOTH AND UNVIORM |
| PEGDMA/10% (1000) | PMUV BUTTONS | 60/8/60D | 70 | 45±1 | 98% COATED; URFACE COATED AND SWELLED BY ISOPROPANOL |

5. Methacrylonitrile, methacrylic acid, acrylic acid and acrylamide did not yield good coatings on PMUV. The coating angles measured varied from 69° to 62° for methacrylonitrile. Methacrylic acid coatings gave a contact angle of 66° at 40% concentration. Acrylamide left powder residues on sample surface, after grafting. The contact angle did not show a significant change for this material.

In general, glyceryl monomethacrylate, PEO (polyethylene glycol mono- and dimethacrylate) and HEMA yielded the best results based on appearance and consisting of water contact angle. NVP grafting was easy to characterised by contact angle measurement and FTIR, but did not yield consistent results. Polyethylene glycol 200 dimethacrylate, known for its low protein absorption and low cell adhesion characteristics, is one of the preferred polymers for a photografting PMUV material.

Table VI lists the contact angles measured for the different monomers at various concentrations.

Table VII shows the properties of PEO oligomer grafting on PMUV.

## TABLE VI

### RESULTS OF DIFFERENT MONOMERS/POLYMERS GRAFTED ON PMMA SUBSTRATES

| MONOMER GRAFTED | LOT NO. | CONCENTRATION & SOLVENT | CONTACT ANGLE | NOTES |
|---|---|---|---|---|
| NVP | MP-99-41 | 10% NVP & 0.1% IRG184 IN IPA | 40.6 +/- 11.7 | |
| NVP | MP-99-49 | 5% NVP & 0.1% IRG 184 IN IPA | 44.8 +/- 12.2 | |
| NVP | MP-99-87 | 5% NVP+0.3% IRG 184<br>10% NVP+0.3% IRG 184<br>15% NVP+0.3% IRG 184<br>20% NVP+0.3% IRG 184 | 66.4 +/- 0.9<br>64.8 +/- 1.1<br>41.1 +/- 6.6<br>39.8 +/- 12.2 | CRAZING ON SAMPLE SURFACE |
| METHACRYLON-ITRILE | MP-99-135 | 5% MACN+0.3% IRG 184<br>10% MACN+0.3% IRG 184<br>20% MACN+0.3% IRG 184<br>40% MACN+0.3% IRG 184 | 69.2 +/- 2.5<br>67.0 +/- 0.0<br>67.5 +/- 1.3<br>66.0 +/- 2.0 | |
| METHACRYLON-ITRILE | MP-99-147 | 60% MACN+0.3% IRG 184<br>80% MACN+0.3% IRG 184 | 65.5 +/- 3.0<br>62.8 +/- 2.2 | |
| HEMA | MP-99-118 | 5% HEMA + 0.1% IRG 184<br>10% HEMA + 1% IRG 184<br>15% HEMA + .1% IRG 184<br>20% HEMA + .1% IRG 184 | 66.3 +/- 3.5<br>64.7 +/- 2.9<br>64.7 +/- 2.9<br>63.0 +/- 1.0 | |
| HEMA | MP-99-121 | 10% HEMA + .2% IRG 184<br>20% HEMA + .2% IRG 184<br>40% HEMA + .2% IRG 184<br>80% HEMA + .2% IRG 184 | 64.0 +/- 0.0<br>62.0 +/- 0.0<br>60.0 +/- 0.0<br>58.7 +/- 2.5 | |
| HEMA | MP-99-125 | 10% HEMA + .2% IRG 184<br>20% HEMA + .2% IRG 184<br>40% HEMA + .2% IRG 184<br>80% HEMA + .2% IRG 184<br>SOLVENT:<br>ACETONE: IPA<br>(4:6) | 65.5 +/- 5.0<br>64.0 +/- 0.0<br>62.3 +/- 3.5<br>53.8 +/- 6.7 | |

| HEMA | MP-99-143 | 60% HEMA + .2% IRG 184<br>SOLVENT:<br>ACETONE: IPA<br>(4:6) | 53.8 +/- 5.5 | |
|---|---|---|---|---|
| GLYCERYL MONOMETHACRYLATE | MP-99-123 | 10% GMMA+.1% IRG 184<br>20% GMMA+.1% IRG 184 | 63.8 +/- 1.5<br>49.5 +/- 14.4 | |
| GLYCERYL MONOMETHACRYLATE | MP-99-127 | 10% GMMA+.1% IRG 184<br>20% GMMA+.1% IRG 184<br>40% GMMA+.1% IRG 184<br>80% GMMA+.1% IRG 184<br>SOLVENT: IPA | 61.3 +/- 2.3<br>54.8 +/- 7.3<br>49.5 +/- 9.1<br>36.0 +/- 11.2 | |
| GLYCERYL MCNOMETHACRYLATE | MP-88-145 | 10% GMMA+.1% IRG 184<br>20% GMMA+.1% IRG 184<br>40% GMMA+.1% IRG 184<br>60% GMMA+.1% IRG 184<br>80% GMMA+.1% IRG 184 | 83.8 +/- 2.2<br>59.3 +/- 2.1<br>62.0 +/- 0.0<br>51.5 +/- 5.9<br>36.8 +/- 5.9 | |
| PCLYETHYLENE GLYCOL 200 DIMETHACRYLATE | MP-99-148 | 40% PEGDMA+.1% IRG 184 | 53.3 +/- 4.2 | |
| POLYETHYLENE GLYCOL 200 DIMETHACRYLATE | MP-99-137 | 5% PEGDMA+.1% IRG 184<br>10% PEGDMA+.1% IRG 184<br>20% PEGDMA+.1% IRG 184<br>40% PEGDMA+.1% IRG 184 | 65.5 +/- 1.0<br>67.8 +/- 7.4<br>71.3 +/- 6.3<br>71.5 +/- 3.1 | |
| METHACRYLIC ACID | MP-99-131 | 5% MA+.1% IRG 184<br>10% MA+.1% IRG 184 | 66.0 +/- 0.0<br>66.0 +/- 0.0 | |
| METHACRYLIC ACID | MP-99-133 | 20% MA+.1% IRG 184<br>40% MA+.1% IRG 184 | 69.0 +/- 0.0<br>65.8 +/- 1.5 | |
| ACRYLIC ACID | MP-99-139 | 5% AA+.1% IRG 184<br>10% AA+.1% IRG 184<br>20% AA+.1% IRG 184<br>40% AA+.1% IRG 184 | 66.0 +/- 2.5<br>72.8 +/- 2.6<br>71.0 +/- 0.0<br>70.7 +/- 0.6 | |
| ACRYLAMIDE | MP-99-129 | 5% AC+.1% IRG 184<br>10% AC+.1% IRG 184 | 61.5 +/- 10.7<br>67.2 +/- 11.7 | |

## TABLE VII

## RESULTS OF PEO OLIGOMER GRAFTING ON PMUV

| OLIGOMER/CONC. (%) | SUBSTRATE | SOAKING/IRRADIATING/ WASHING TIME (MIN.) | THICKNESS OF GRAFTING (UM) | CONTACT ANGLES | COMMENTS |
|---|---|---|---|---|---|
| PEGDA 259/100 | PMUV BUTTONS | 60/5/60 IN IPA | 230 | 59-63 | SURFACE LOOKED SMOOTH AND UNIFORM, ABOUT 100% COATED |
| PEGDA 259/60 IN MeOH | PMUV BOTTONS | 60/5/60 IN MeOH | 100 | 61-64 | SURFACE LOOKED SMOOTH AND UNIFORM, ABOUT 90% COATED |
| PEGDA 259/80 IN MeOH | PMUV BUTTONS | 60/5/60 IN MeOH | 160 | 60 64 | SURFACE LOOKED SMOOTH AND THICKER THAN THAT OF 60%, ABOUT 92% COATED. |
| PEGDA 259/80 IN MeOH | PMUV BUTTONS | 60/8/60 IN MeOH | 100 | 61-64 | POLYMERIZATION OCCURED MORE COMPLETELY THAN THAT OF 5 MINUTES IRRADIATED |
| PEGDA 210/80 IN MeOH | PMUV BUTONS | 60/8/60 IN MeOH | 100 | 60-64 | SURFACE LOOKED SMOOTHER THAN THAT OF PEGDA 259 |
| PEGDA 1000/10 IN MeOH | PMUV BUTTONS | 60/8/60 IN IPA | 70 | 43-46 | SURFACE CRACKED AND SWELLED AFTER IPA RINSE |
| PEGDA 252/10 IN MeOH | PMUV BUTTONS | 60/20/60 IN MeOH | 10 | 55-65 | SURFACE SWELLED GREATLY IN MeOH, ABOUT 50% COATED |
| PEGDA 252/20 IN MeOH | PMUV BUTTONS | 60/8/30 IN IPA | 16 | 45 50 | SURFACE LOOKED SMOOTH AND UNIFORM, ABOUT 100% COATED, SLIGHTLY SWELLED IN IPA |
| PEGDA 252/20 IN MeOH | PMUV BUTTONS | 60/8/30 IN IPA | 10 | 45-50 | SURFACE LOOKED SMOOTH AND UNIFORM, NOT SWELLED IN IPA, ABOUT 100% COATED |
| PEGDA 252/20 IN MeOH | PMUV BUTTONS | 30/8/30 IN IPA | 10 | 45 50 | SURFACE LOOKED AS SMOOTH AND UNIFORM AS THAT OF 60 MINUTE SOAKING, NOT SWELLED IN IPA |

14

**Claims**

1. A polymerizable coating composition comprising a hydrophilic monomer and a hydrophobic monomer comprising perfluoroacrylate or perfluoromethacrylate.

2. A composition according to claim 1 wherein the hydrophilic monomer comprises acrylamide, hydroxyethylmethacrylate, hydroxyethyacrylate, hydroxypropylmethacrylate, hydroxypropylacrylate or vinyl pyrrolidone.

3. A composition according to claim 1 or claim 2 wherein the molar ratio of hydrophilic to hydrophobic monomer is from about 1:1 to about 1:5.

4. An article having a surface coated with a polymeric composition comprising residues derived from a hydrophilic monomer and residues derived from perfluoroacrylate or perfluoromethacrylate.

5. An article according to claim 4 in which the polymeric composition is grafted onto the surface of the article.

6. An article according to claim 4 or claim 5 wherein the flexible article is made of rubber.

7. An article according to claim 6 wherein the rubber is natural rubber.

8. An article according to claim 7 wherein rubber is partly vulcanized.

9. An article according to any one of claims 4 to 8 wherein the article is a surgical or examination glove, a catheter, a condom or a ureter.

10. An article according to claim 4 or claim 5 wherein the article is made from an acrylic resin.

11. An article according to claim 10 wherein the resin is polymethylmethacrylate.

12. An article according to claims 4, 10 or 11 in the form of a contact lens or an intraocular lens.

13. A process for the manufacture of articles having a coated surface which comprises coating said surface with a polymerizable composition comprising hydrophilic monomers and hydrophobic monomers and thereafter subjecting the composition to irradiation to polymerize the composition, which the hydrophobic monomers comprise a perfluoroacrylic or a perfluoromethacrylate.

14. A process according to claim 13 wherein the irradiation is ultraviolet light irradiation.

15. A solution suitable for photografting comprised of low molecular weight polymers of ethylene oxide terminated with one or two polymerizable groups and a photocuring inhibitor capable of absorbing ultraviolet light.

16. A solution suitable for photografting according to claim 15, in which the polymer of ethylene oxide has a molecular weight not less than 200 and not more than 5,000.

17. A method of forming an article having a surface coated with a polymeric composition comprising residues derived from a hydrophilic monomer and residues derived from perfluoroacrylate or perfluoromethacrylate, comprising dipping said article into a solution suitable for photografting comprised of low molecular weight polymers of ethylene oxide terminated with one or two polymerizable groups and a photocuring inhibitor capable of absorbing ultraviolet light prior to subjecting the article to said ultraviolet light.

18. An intraocular lens comprised of an acrylic copolymer having a banded coating of the photografted solution of claim 16.